# EUROPEAN PATENT APPLICATION

(11) **EP 4 520 760 A1**
(43) Date of publication of application: **12.03.2025**
(21) Application number: 22936368.4
(22) Date of filing: 15.11.2022
(51) Int. Cl.: C07D 515/22, A61K 31/4995, A61P 35/00

(54) **LURBINECTEDIN CRYSTAL FORM AND METHOD FOR PREPARING SAME**

(30) Priority: 08.04.2022 CN 202210369453; 01.09.2022 CN 202211063012
(71) Applicant: Shanghai Haoyuan Chemexpress Co., Ltd., Shanghai 201203 (CN)
(72) Inventor: ZHOU, Houjia, Shanghai 201203 (CN); QIAN, Kaimin, Shanghai 201203 (CN); CAI, Lingli, Shanghai 201203 (CN); GAO, Qiang, Shanghai 201203 (CN); ZHENG, Baofu, Shanghai 201203 (CN)
(74) Representative: Potter Clarkson
(86) International application number: PCT/CN2022/131995
(87) International publication number: WO 2023/193440

(57) **Abstract**

Provided is a lurbinectedin crystal form having characteristic absorption peaks at diffraction angles 2θ of 4.55± 0.2°, 6.16 ± 0.2°, 7.61 ±0.2°, 9.17 ±0.2°, 9.54 ±0.2°, and 9.89±0.2° in an X-ray powder diffraction pattern using a Cu-Kα radiation source. Also provided are novel lurbinectedin crystal forms II, III, IV, and V. The crystal forms I-V features high purity, good stability, good solubility and the like, the absence of the problems of difficulties in filtration and static electricity in the prior art, a simple preparation method, available and cost-efficient solvents, mild crystallization conditions, and suitability for industrial mass production.

## Description

This application claims priority to Chinese Patent Application No. 202210369453.6, which is submitted to the China National Intellectual Property Administration with the title of "LURBINECTEDIN CRYSTAL FORM AND METHOD FOR PREPARING SAME", filed on April 08, 2022, and Chinese Patent Application No. 202211063012.X, which is submitted to China National Intellectual Property Administration with the title of "LURBINECTEDIN CRYSTAL FORM AND METHOD FOR PREPARING SAME", filed on September 01, 2022; these applications are incorporated herein by reference in their entireties.

### TECHNICAL FIELD

The present invention relates to a drug crystal form and method for preparing same, specifically relates to crystal form of lurbinectedin and method for preparing same.

### BACKGROUND

Lurbinectedin--On June 15, 2020, U.S. FDA approved the marketing of lurbinectedin lyophilized powder for injection, for intravenous use, with brand name of Zepzelca^{®}. Zepzelca^{®} (Lurbinectedin) is the first new drug approved for second-line therapy of small cell lung cancer (SCLC) since 1996, used for treatment in adult patients with metastatic small cell lung cancer who have progressed following prior platinum-based chemotherapy.

CN114940682A discloses an X-ray powder diffraction pattern of the crystal form A of lurbinectedin (Form A) having characteristic absorption peaks at diffraction angles 2θ of 5.020±0.20, 9.279±0.20, 9.701±0.20, 13.222±0.20 and 19.235±0.20, and used dichloromethane, chloroform or carbon tetrachloride as crystallization solvent; wherein chloroform or carbon tetrachloride are highly-toxic reagents and should be avoided; in addition, our experimental study found that the crystal Form A is stable at -20°C but highly unstable at room temperature, and converts into other crystal forms readily.

Crystal form patent WO2021099635 discloses the crystal Form B of lurbinectedin, which X-ray powder diffraction pattern having characteristic absorption peaks at diffraction angles 20 of 6.2±0.2°, 7.6±0.2°, 9.0±0.2°, 10.9±0.2°, 14.9±0.2° and 15.3±0.2°. This patent named the pure lurbinectedin compound obtained through chromatographic treatment in the disclosed prior art WO2003014127 as Form A and pointed out that this Form A of lurbinectedin is amorphous and has static electricity during its manipulation. In many pharmaceutical operations, uncontrolled static electricity can lead to significant production challenges. It comprises the problems of product contamination, material loss, cleaning difficulties and safety hazards, while the current polymorph screening of lurbinectedin still remains inadequate; therefore, there is a need for further screening to find a more suitable crystal form for drug development.

### SUMMARY OF INVENTION

The technical problem to be solved by the present invention is to overcome the defects of the crystal form of lurbinectedin in the prior art, and thus a new crystal form of lurbinectedin and method for preparing same are provided. The lurbinectedin crystal form of the present invention has excellent stability, simple preparation method, suitability for industrial mass production, and promising prospect for further drug development.

The present invention relates to new crystal forms of lurbinectedin, and the chemical formula of lurbinectedin is as shown in Formula (I):

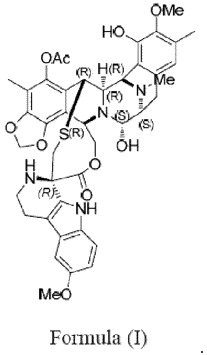

The present invention provides a crystal form I of lurbinectedin, having characteristic absorption peaks at diffraction angles 20 of 4.55±0.2°, 6.16±0.2°, 7.61± 0.2°, 9.17±0.2°, 9.54±0.2°, and 9.89±0.2° in an X-ray powder diffraction pattern using a Cu-Kα radiation source.

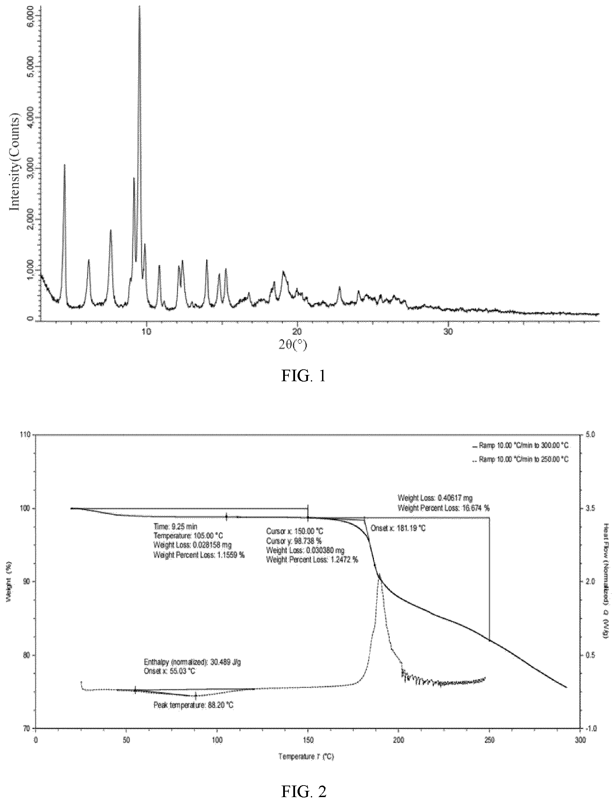

The present invention provides a crystal form I of lurbinectedin, having one or more of the following characteristics:
1) the crystal form I of lurbinectedin has characteristic absorption peaks at diffraction angles 2θ of 4.55±0.2°, 6.16±0.2°, 7.61±0.2°, 9.17±0.2°, 9.54±0.2°, and 9.89±0.2° in the X-ray powder diffraction pattern, and has one or more of the characteristic peaks at diffraction angles 2θ of: 8.94±0.2°, 10.84±0.2°, 11.16±0.2°, 12.15±0.2°, 12.38±0.2°, 13.00±0.2°, 13.98±0.2°, 14.80±0.2°, 15.24±0.2°, 16.15±0.2°, 16.79±0.2°, 18.36±0.2°, 19.09±0.2°, 19.96±0.2°, 20.53±0.2°, 21.17±0.2°, 22.78±0.2°, 24.04±0.2°, 24.56±0.2°, 25.51±0.2°, 25.91±0.2°, 26.40±0.2° and 27.09±0.2°;
2) the X-ray powder diffraction pattern of the crystal form I is substantially as shown in FIG.1; and
3) the crystal form I has a DSC-TGA thermogram substantially as shown in FIG.2. The present invention provides a crystal form I of lurbinectedin, wherein the crystal form I has characteristic absorption peaks at diffraction angles 2θ of 4.55±0.2°, 6.16±0.2°, 7.61±0.2°, 8.94±0.2°, 9.17±0.2°, 9.54±0.2°, 9.89±0.2°, 10.84±0.2°, 12.15±0.2° and 12.38±0.2° in the X-ray powder diffraction pattern using a Cu-Kα radiation source.

The present invention provides a crystal form I of lurbinectedin, wherein the crystal form I has characteristic absorption peaks at diffraction angles 2θ of 4.55±0.2°, 6.16±0.2°, 7.61±0.2°, 8.94±0.2°, 9.17±0.2°, 9.54±0.2°, 9.89±0.2°, 10.84±0.2°, 12.15±0.2°, 12.38±0.2°, 13.98±0.2°, 14.80±0.2°, 15.24±0.2°, 19.09±0.2° and 22.78±0.2° in the X-ray powder diffraction pattern using a Cu-Kα radiation source. The present invention provides a crystal form I of lurbinectedin, wherein the crystal form I has characteristic absorption peaks at diffraction angles 2θ of 4.55± 0.2°,6.16±0.2°, 7.61±0.2°, 8.94±0.2°, 9.17±0.2°, 9.54±0.2°, 9.89±0.2°, 10.84±0.2°, 11.16±0.2°, 12.15±0.2°, 12.38±0.2°, 13.00±0.2°, 13.98±0.2°, 14.80±0.2°, 15.24±0.2°, 16.15±0.2°, 16.79±0.2°, 18.36±0.2°, 19.09±0.2°, 19.96±0.2°, 20.53±0.2°, 21.17±0.2°, 22.78±0.2°, 24.04±0.2°, 24.56±0.2°, 25.51±0.2°, 25.91±0.2°, 26.40±0.2° and 27.09±0.2° in the X-ray powder diffraction pattern using a Cu-Kα radiation source. Without limitation, in one specific embodiment of the present invention, the X-ray powder diffraction pattern of crystal form I of lurbinectedin is as shown in FIG.1. Without limitation, in one specific embodiment of the present invention, the crystal form I of lurbinectedin provided by the present invention when subjected to thermogravimetric analysis, exhibits a weight loss of about 5% or less, about 4% or less, about 3% or less, preferably about 2% or less, and more preferably around 1.2%-1.5%; significant weight loss begins within the temperature range of 150-250°C; and in one specific embodiment, the crystal form I exhibits significant weight loss at peak point of about 181°C. The total weight loss within 150-250°C is about 20% or less, about 18% or less, preferably about 17% or less, more preferably the mass loss is about 16-17%, and the DSC-TGA thermogram is as shown in FIG.2.

The present invention also provides a method for preparing the crystal form I of lurbinectedin, comprising the following step:
1) dissolving lurbinectedin in a good solvent at an appropriate temperature, then adding an anti-solvent dropwise until turbidity appears, followed by stirring at a certain temperature to induce crystallization, filtering, and drying to obtain the crystal form I; or
2) dissolving lurbinectedin in a mixed solvent of a good solvent and an anti-solvent at an appropriate temperature, stirring to induce crystallization, and filtering and drying the resultant to obtain the crystal form I.

The good solvent is preferably an aprotic polar solvent, which is one or more member selected from the group consisting of N-methyl pyrrolidone, nitrile solvent and ketone solvent; preferably, the nitrile solvent is selected from the group consisting of acetonitrile, propionitrile, butyronitrile and isobutyronitrile; preferably, the ketone solvent is selected from the group consisting of acetone, butanone and 2-pentanone; more preferably, the aprotic polar solvent is one or more member selected from the group consisting of N-methyl pyrrolidone, acetonitrile, propionitrile, butyronitrile, isobutyronitrile, acetone, butanone and 2-pentanone, and especially more preferably is acetonitrile or acetone.

The anti-solvent is preferably one or more member selected from the group consisting of water and an ether solvent, more preferably one or more member selected from the group consisting of water, diethyl ether, isopropyl ether and methyl tert-butyl ether, especially more preferably water.

The volume ratio of good solvent to the anti-solvent is preferably 1:3-3:1, more preferably 1:1-2:1.

The mass-to-volume ratio of lurbinectedin to the solvent can be selected according to conventional methods in the art, preferably 1 g : 5 mL - 1 g : 30 mL, more preferably 1 g : 10 mL - 1 g : 20 mL, wherein the solvent is a generic term for the good solvent and the anti-solvent.

In some embodiments, the appropriate temperature can be selected according to the conventional range in the art, preferably 10-25°C.

In some embodiments, the time of stirring to induce crystallization can be selected according to the conventional range in the art, preferably 0.5-10 hours, more preferably 0.5-2 hours.

Preferably, after crystallization, the filtering and drying are performed according to procedures known in the art. The filtering may be selected from conventional operations in the art, preferably suction filtration, pressure filtration or cake filtration, more preferably cake filtration. After the cake filtration is finished, preferably, the filter cake is washed with an anti-solvent. The drying is selected from conventional operations in the art, preferably vacuum drying at 25°C.

The present invention also provides a crystal form II of lurbinectedin, which is an anhydrous crystal form.

The present invention provides a crystal form II of lurbinectedin, having characteristic absorption peaks at diffraction angles 2θ of 4.56±0.2°, 9.26±0.2°, 9.54±0.2°, 9.93±0.2°, 13.99±0.2°, 18.46±0.2° and 19.02±0.2° in the X-ray powder diffraction pattern using a Cu-Kα radiation source.

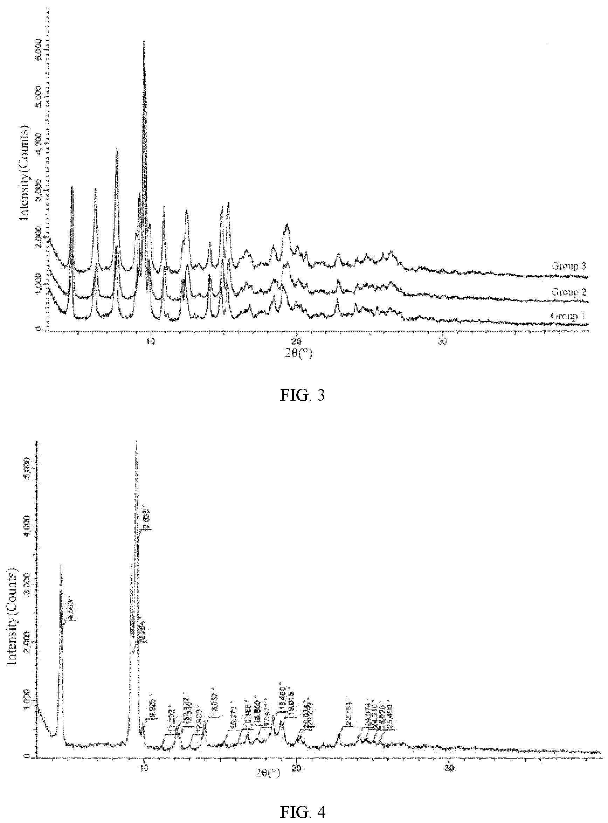

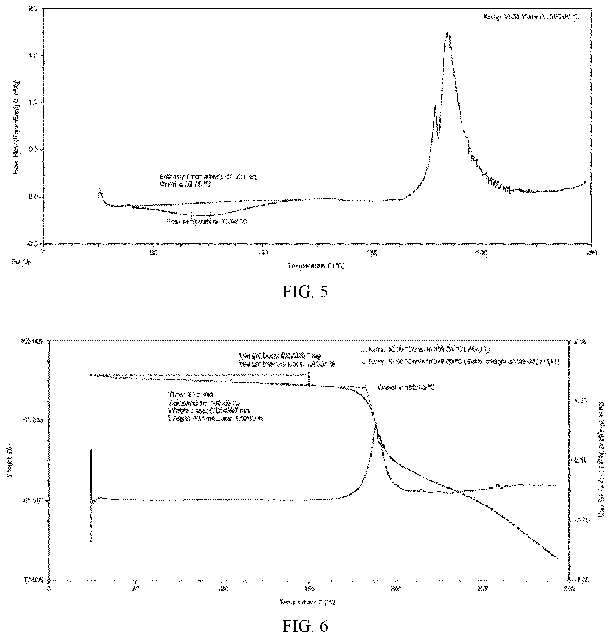

The present invention provides a crystal form II of lurbinectedin, having one or more of the following characteristics:
1) the crystal form II of lurbinectedin has characteristic absorption peaks at diffraction angles 2θ of 4.56±0.2°, 9.26±0.2°, 9.54±0.2°, 9.93±0.2°, 13.99±0.2°, 18.46±0.2° and 19.02±0.2° in the X-ray powder diffraction pattern, and has one or more of the characteristic peaks at diffraction angles 2θ of: 11.20±0.2°, 12.13±0.2°, 12.34±0.2°, 12.99±0.2°, 15.27±0.2°, 16.19±0.2°, 16.80±0.2°, 17.41±0.2°, 20.01±0.2°, 20.26±0.2°, 22.78±0.2°, 24.07±0.2°, 24.51±0.2°, 25.02±0.2° and 25.49±0.2°;
2) the X-ray powder diffraction pattern of the crystal form II is substantially as shown in FIG.4;
3) the crystal form II has a DSC thermogram substantially as shown in FIG.5; and
4) the crystal form II has a TGA thermogram substantially as shown in FIG.6.

The present invention provides a crystal form II of lurbinectedin, having characteristic absorption peaks at diffraction angles 2θ of 4.56±0.2°, 9.26±0.2°, 9.54±0.2°, 9.93±0.2°, 12.13±0.2°, 12.34±0.2°, 13.99±0.2°, 18.46±0.2°, 19.02±0.2° and 22.78±0.2° in the X-ray powder diffraction pattern using a Cu-Kα radiation source. The present invention provides a crystal form II of lurbinectedin, having characteristic absorption peaks at diffraction angles 2θ of 4.56±0.2°, 9.26±0.2°, 9.54±0.2°, 9.93±0.2°, 11.20±0.2°, 12.13±0.2°, 12.34±0.2°, 12.99±0.2°, 13.99±0.2°, 15.27±0.2°, 16.19±0.2°, 16.80±0.2°, 17.41±0.2°, 18.46±0.2°, 19.02±0.2°, 20.01±0.2°, 20.26±0.2°, 22.78±0.2°, 24.07±0.2°, 24.51±0.2°, 25.02±0.2° and 25.49±0.2° in the X-ray powder diffraction pattern using a Cu-Kα radiation source.

Without limitation, in one specific embodiment of the present invention, the X-ray powder diffraction pattern of the crystal form II of lurbinectedin is as shown in FIG.4.

Without limitation, in one specific embodiment of the present invention, the crystal form II of lurbinectedin provided by the present invention, when subjected to differential scanning calorimetry (DSC) with a heating rate of 10°C/min from room temperature to 250°C, exhibits a broad endothermic peak between 25-100°C, and two obvious exothermic peaks in the temperature range of 150-200°C; corresponding to thermogravimetric analysis (TGA), from room temperature to about 150°C, the weight loss is about 3% or less, about 2.5% or less, preferably about 2% or less, more preferably 1.5% or less; decomposition and weight loss begin in the temperature range of 150-200°C, in one specific embodiment, the peak point is about 183°C; the DSC thermogram is as shown in FIG.5, the TGA thermogram is as shown in FIG.6.

The present invention also provides a method for preparing the crystal form II of lurbinectedin, comprising the following steps:
1) adding lurbinectedin to the solvent and suspending at an appropriate temperature for a certain time;
2) filtering; and
3) drying at a certain temperature to obtain the crystal form II.

The solvent can be a single solvent or a mixed solvent, including but not limited to one or more of nitriles, esters, chlorinated alkanes and ethers, more preferably acetonitrile, dichloromethane or a mixed solvent of acetonitrile and ethyl acetate. The appropriate temperature is room temperature or high temperature, preferably 20-50°C; the suspension time is 12 hours-4 days, preferably 1-4 days, more preferably 4 days; the filtration is any of the solid-liquid separation methods such as centrifugal filtration and vacuum filtration; the drying is vacuum drying or blast drying, wherein the temperature is room temperature or high temperature, preferably 20-50°C, and more preferably the drying is a vacuum drying at 40-50°C;

The mass-to-volume ratio of lurbinectedin to the solvent is 40:1 mg:mL - 75:1 mg:mL.

The present invention also provides a method for preparing the crystal form II of lurbinectedin, comprising the following steps:
1) dissolving lurbinectedin in a good solvent at an appropriate temperature;
2) adding an anti-solvent to the solution obtained in 1);
3) stirring to induce crystallization at a certain temperature;
4) filtering; and
5) drying to obtain the crystal form II.

The good solvent is selected from haloalkane solvent, preferably dichloromethane or chloroform; the anti-solvent is a nitrile solvent, preferably acetonitrile, and the anti-solvent is used to reduce the solubility to precipitate solid. The appropriate temperature is 20-25°C; the certain temperature can be selected according to the conventional range in the art, preferably 15-35°C, further preferably 20-25°C; the volume ratio of the good solvent to the anti-solvent is 1:(6-9); the mass-to-volume ratio of Lurbinectedin to the total volume of the good solvent and the anti-solvent is 1:(2-4) mg:mL.

The present invention also provides a crystal form III of lurbinectedin, which is a hydrate crystal form.

The present invention provides a crystal form III of lurbinectedin, having characteristic absorption peaks at diffraction angles 20 of 4.82±0.2°, 8.75±0.2°, 10.13±0.2°, 10.51±0.2°, 13.23±0.2° and 17.98±0.2° in the X-ray powder diffraction pattern using a Cu-Kα radiation source.

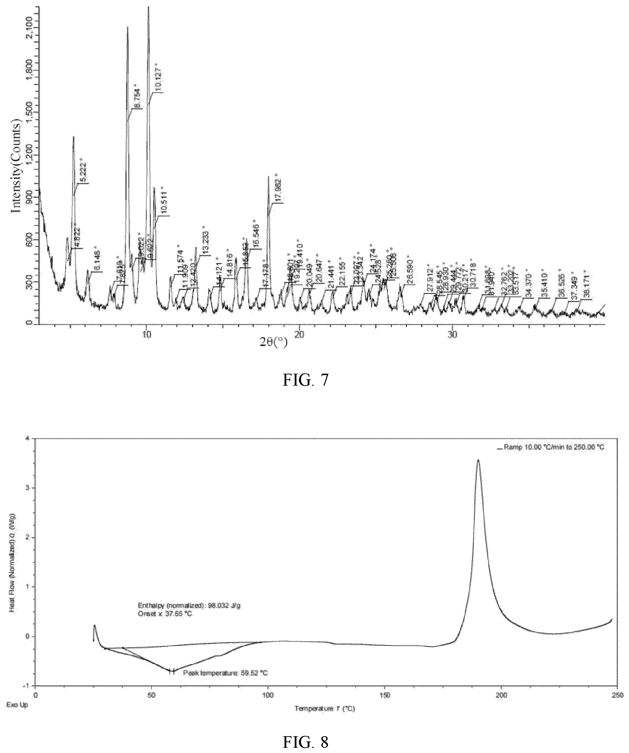

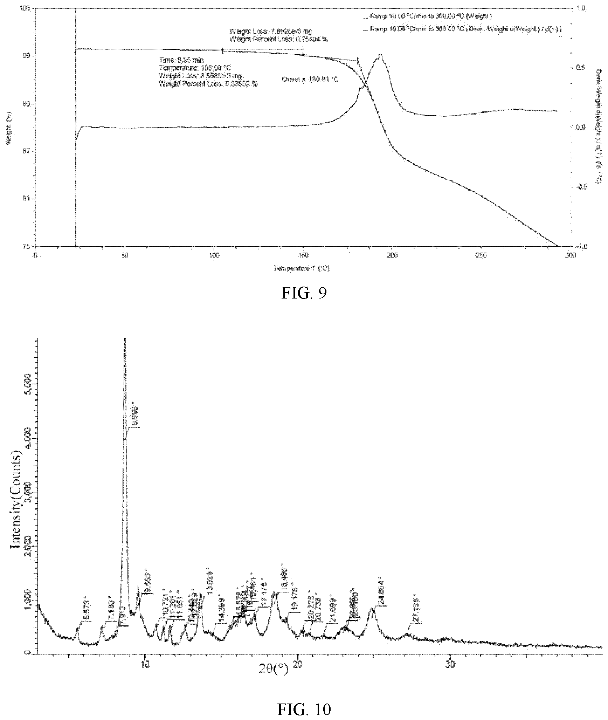

The present invention provides a crystal form III of lurbinectedin, having one or more of the following characteristics:
1) the crystal form III of lurbinectedin has characteristic absorption peaks at diffraction angles 2θ of 4.82±0.2°, 8.75±0.2°, 10.13±0.2°, 10.51±0.2°, 13.23±0.2° and 17.98±0.2° in the X-ray powder diffraction pattern, and has one or more of the characteristic peaks at diffraction angles 2θ of: 5.22±0.2°, 6.15±0.2°, 7.62±0.2°, 7.83±0.2°, 9.02±0.2°, 9.62±0.2°, 11.57±0.2°, 11.91±0.2°, 12.42±0.2°, 14.12±0.2°, 14.82±0.2°, 15.85±0.2°, 16.55±0.2°, 17.18±0.2°, 18.8±0.2°, 19.21±0.2°, 19.41±0.2°, 20.65±0.2°, 23.10±0.2°, 23.34±0.2°, 24.17±0.2°, 25.26±0.2° and 25.51±0.2°;
2) the X-ray powder diffraction pattern of the crystal form III is substantially as shown in FIG.7;
3) the crystal form III has a DSC thermogram substantially as shown in FIG.8; and
4) the crystal form III has a TGA thermogram substantially as shown in FIG.9.

The present invention provides a crystal form III of lurbinectedin, having characteristic absorption peaks at diffraction angles 20 of 4.82±0.2°, 8.75±0.2°, 9.02±0.2°, 9.62±0.2°, 10.13±0.2°, 10.51±0.2°, 13.23±0.2°, 15.85±0.2°, 16.55±0.2° and 17.98±0.2° in the X-ray powder diffraction pattern using a Cu-Kα radiation source.

The present invention provides a crystal form III of lurbinectedin, having characteristic absorption peaks at diffraction angles 20 of 4.82±0.2°, 5.22±0.2°, 6.15±0.2°, 8.75±0.2°, 9.02±0.2°, 9.62±0.2°, 10.13±0.2°, 10.51±0.2°, 11.57±0.2°, 13.23±0.2°, 14.12±0.2°, 14.82±0.2°, 15.85±0.2°, 16.55±0.2°, 17.98±0.2° and 19.41±0.2° in the X-ray powder diffraction pattern using a Cu-Kα radiation source.

The present invention provides a crystal form III of lurbinectedin, having characteristic absorption peaks at diffraction angles 20 of 4.82±0.2°, 5.22±0.2°, 6.15±0.2°, 7.62±0.2°, 7.83±0.2°, 8.75±0.2°, 9.02±0.2°, 9.62±0.2°, 10.13±0.2°, 10.51±0.2°, 11.57±0.2°, 11.91±0.2°, 12.42±0.2°, 13.23±0.2°, 14.12±0.2°, 14.82±0.2°, 15.85±0.2°, 16.55±0.2°, 17.18±0.2°, 17.98±0.2°, 18.8±0.2°, 19.21±0.2°, 19.41±0.2°, 20.65±0.2°, 23.10±0.2°, 23.34±0.2°, 24.17±0.2°, 25.26±0.2° and 25.51±0.2° in the X-ray powder diffraction pattern using a Cu-Kα radiation source.

Without limitation, in one specific embodiment of the present invention, the X-ray powder diffraction pattern of the crystal form III of lurbinectedin is as shown in FIG.7.

Without limitation, in one specific embodiment of the present invention, the crystal form III of lurbinectedin provided by the present invention, when subjected to differential scanning calorimetry (DSC) with a heating rate of 10°C/min from room temperature to 250°C, exhibits a broad endothermic peak between 25-100°C and obvious exothermic peaks between about 175-200°C; corresponding to thermogravimetric analysis, from room temperature to 105°C, the weight loss is about 1.5% or less, preferably about 1% or less, more preferably a weight loss of 0-0.5%; from room temperature to about 150°C, the weight loss is about 3% or less, about 2% or less, about 1.5% or less, preferably about 1% or less, more preferably a weight loss of 0-0.8%. Decomposition and weight loss begin in the temperature range of 150-200°C, and in one specific embodiment, the peak point is about 181°C. The DSC thermogram is as shown in FIG.8, and the TGA thermogram is as shown in FIG.9.

The present invention also provides a method for preparing the crystal form III of lurbinectedin, comprising the following steps:
1) dissolving lurbinectedin in a good solvent at an appropriate temperature;
2) adding an anti-solvent to the solution obtained in 1);
3) stirring to induce crystallization;
4) filtering; and
5) drying at room temperature to obtain the crystal form III.

The good solvent is preferably an aprotic polar solvent, preferably an ether solvent; more preferably tetrahydrofuran (THF).

The anti-solvent is preferably one or more of water or alkane solvent, and the alkane solvent is preferably one or more member selected from the group consisting of n-pentane, n-hexane, n-heptane, and n-octane, more preferably is n-heptane.

The volume ratio of good solvent to the anti-solvent is preferably 1:10-10:1, more preferably 1:2-1:10.

The mass-to-volume ratio of lurbinectedin to the good solvent can be selected according to conventional methods in the art, preferably 1 g : 60 mL - 1 g : 500 mL, more preferably 1 g : 60 mL - 1 g : 200mL.

In some embodiments, the appropriate temperature can be selected according to the conventional range in the art, preferably select from 20-25°C.

Preferably, after crystallization, the filtering and drying are performed according to procedures known in the art. The filtration is selected from conventional operations in the art, preferably suction filtration, pressure filtration or cake filtration, more preferably cake filtration. After the cake filtration is finished, preferably, the filter cake is washed with an anti-solvent. The drying may be performed by conventional operations in the art, preferably drying at room temperature.

The present invention also provides a method for preparing the crystal form III of lurbinectedin, comprising the following steps:
1) adding lurbinectedin to a mixed solvent at an appropriate temperature;
2) stirring to induce crystallization;
3) filtering; and
4) drying to obtain the crystal form III.

The mixed solvent includes but not limited to a mixed solvent of ethers and water, or a mixed solvent of esters and alcohols, especially more preferably a mixed solvent of tetrahydrofuran and water, dioxane and water, or ethyl acetate and isopropanol. In the mixed solvent of ethers and water, the volume ratio of ethers to water is 1:(0.8-1.2); in the mixed solvent of esters and alcohols, the volume ratio of esters to alcohols is 1:(0.8-1.2).

In some embodiments, the appropriate temperature can be selected according to the conventional range in the art, preferably 25-50°C; the mass-to-volume ratio of lurbinectedin to the mixed solvent is 1:(18-22) g:mL.

The present invention also provides a crystal form IV of lurbinectedin, which is an anhydrous crystal form.

The present invention provides a crystal form IV of lurbinectedin, having characteristic absorption peaks at diffraction angles 2θ of 5.57±0.2°, 7.18±0.2°, 8.70±0.2°, 11.20±0.2°, 11.65±0.2° and 13.63±0.2° in the X-ray powder diffraction pattern using a Cu-Kα radiation source.

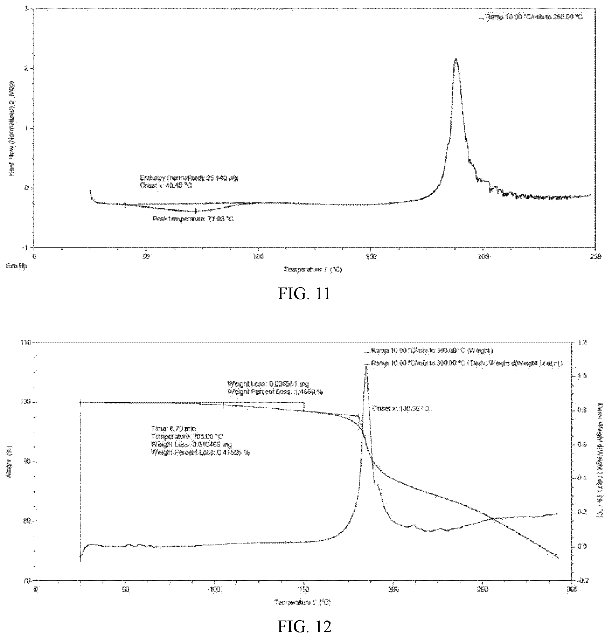

The present invention provides a crystal form IV of lurbinectedin, having one or more of the following characteristics:
1) the crystal form IV of lurbinectedin having characteristic absorption peaks at diffraction angles 20 of 5.57±0.2°, 7.18±0.2°, 8.70±0.2°, 11.20±0.2°, 11.65±0.2° and 13.63±0.2° in the X-ray powder diffraction pattern, and has one or more of the characteristic peaks at diffraction angles 2θ of: 7.91±0.2°, 9.56±0.2°, 10.72±0.2°, 12.44±0.2°, 12.63±0.2°, 14.40±0.2°, 15.58±0.2°, 15.90±0.2°, 16.23±0.2°, 16.46±0.2°, 17.18±0.2°, 18.47±0.2°, 19.18±0.2°, 20.28±0.2°, 20.73±0.2°, 21.70±0.2°, 23.00±0.2°, 23.18±0.2°, 24.86±0.2° and 27.14±0.2°;
2) the X-ray powder diffraction pattern of the crystal form IV is substantially as shown in FIG.10;
3) the crystal form IV has a DSC thermogram substantially as shown in FIG.11; and
4) the crystal form IV has a TGA thermogram substantially as shown in FIG.12.

The present invention provides a crystal form IV of lurbinectedin, having characteristic absorption peaks at diffraction angles 20 of 5.57±0.2°, 7.18±0.2°, 8.70±0.2°, 9.56±0.2°, 10.72±0.2°, 11.20±0.2°, 11.65±0.2°, 13.63±0.2° and 24.86±0.2° in the X-ray powder diffraction pattern using a Cu-Kα radiation source.

The present invention provides a crystal form IV of lurbinectedin, having characteristic absorption peaks at diffraction angles 2θ of 5.57±0.2°, 7.18±0.2°, 7.91±0.2°, 8.70±0.2°, 9.56±0.2,6 10.72±0.2,7 11.20±0.2°, 11.65±0.2°, 12.44±0.2°, 12.63±0.2°, 13.63±0.2°, 14.40±0.2°, 15.58±0.2°, 15.90±0.2°, 16.23±0.2°, 16.46±0.2°, 17.18±0.2°, 18.47±0.2°, 19.18±0.2°, 20.28±0.2°, 20.73±0.2°, 21.70±0.2°, 23.00±0.2°, 23.18±0.2°, 24.86±0.2° and 27.14±0.2° in the X-ray powder diffraction pattern using a Cu-Kα radiation source.

Without limitation, in one specific embodiment of the present invention, the X-ray powder diffraction pattern of the crystal form IV of lurbinectedin is as shown in FIG.10.

Without limitation, in one specific embodiment of the present invention, the crystal form IV of lurbinectedin provided by the present invention, when subjected to differential scanning calorimetry (DSC) with a heating rate of 10°C/min from room temperature to 250°C, exhibits a broad endothermic peak between 25-110°C with peak point of about 40°C, and obvious exothermic peaks in the range of about 150-200°C; corresponding to thermogravimetric analysis (TGA), from room temperature to 105°C, the weight loss is about 3% or less, about 2.5% or less, about 2% or less, preferably 1.5% or less, more preferably a weight loss of 0-1%; from room temperature to about 150°C, the weight loss is about 3% or less, about 2.5% or less, preferably about 2% or less, more preferably1.5% or less. Decomposition and weight loss begin in the temperature range of 150-200°C, and in one specific embodiment the peak point is about 181°C. The DSC thermogram is as shown in FIG. 11, and the TGA thermogram is as shown in FIG. 12.

The present invention also provides a method for preparing the crystal form IV of lurbinectedin, comprising the following steps:
1) adding lurbinectedin to the solvent and suspending or slurring at an appropriate temperature for a certain time;
2) filtering; and
3) vacuum drying to obtain the crystal form IV.

The solvent can be a single solvent or a mixed solvent, including but not limited to one or more of ketones, esters, ethers, alcohols, alkanes and water, preferably a ketone solvent or a mixed solvent of ketones with esters, ethers, alcohols, alkanes or water. The ketones is acetone; the esters is one or more member selected from the group consisting of ethyl acetate and isopropyl acetate; the ethers is one or more member selected from the group consisting of methyl tert-butyl ether and tetrahydrofuran; the alcohols is one or more member selected from the group consisting of methanol, ethanol, and isopropanol; the alkanes is one or more member selected from the group consisting of n-heptane, n-hexane, cyclohexane and methyl cyclohexane. The appropriate temperature is 20-25°C. Preferably, the suspending or slurring is performed by adding lurbinectedin to acetone and suspending or slurring at 20-25°C, or by adding lurbinectedin to a mixed solvent of acetone/n-heptane in a volume ratio of (1-3):1 and suspending or slurring at 20-25°C. The certain time of suspending or slurring is 1 hour-4 days, preferably 12 hours-4 days, more preferably 1-4 days, further preferably 4 days; the filtration is any of solid-liquid separation methods such as centrifugal filtration and pressure filtration; the drying is a vacuum drying, preferably, a vacuum drying at 40-50°C;

The mass-to-volume ratio of lurbinectedin to the solvent is 1:(10-150) g:mL.

The present invention also provides a crystal form V of lurbinectedin, the crystal form V is an acetone solvate.

The present inventors have found that, by introducing the following steps in the synthesis of lurbinectedin - preparing the lurbinectedin acetone solvate from crude lurbinectedin -impurities are removed or reduced, and thereby provides a purified lurbinectedin, such as crystal form V of lurbinectedin, and the obtained crystal form V has a high purity. The crystal form V of lurbinectedin may be converted into crystal form IV by desolvation, and the crystal form V is a highly favorable crystal form for impurity removal and purification.

The present invention provides a crystal form V of lurbinectedin, having characteristic absorption peaks at diffraction angles 2θ of 6.35±0.2°, 8.01±0.2°, 9.72±0.2°, 14.23±0.2°, 17.04±0.2° and 20.42±0.2° in the X-ray powder diffraction pattern using a Cu-Kα radiation source.

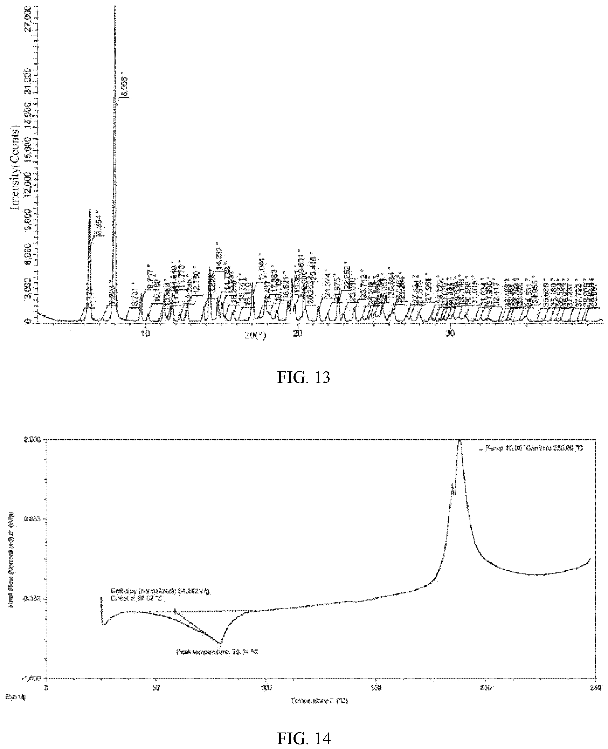

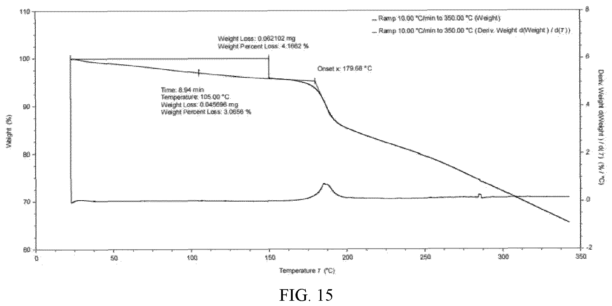

The present invention provides a crystal form V of lurbinectedin, having one or more of the following characteristics:
1) the crystal form V of lurbinectedin has characteristic absorption peaks at diffraction angles 2θ of 6.35±0.2°, 8.01±0.2°, 9.72±0.2°, 14.23±0.2°, 17.04±0.2° and 20.42±0.2° in the X-ray powder diffraction pattern, and has one or more of the characteristic peaks at diffraction angles 20 of: 7.22±0.2°, 10.18±0.2°, 11.25±0.2°, 11.78±0.2°, 12.75±0.2°, 13.82±0.2°, 14.77±0.2°, 15.04±0.2°, 15.74±0.2°, 17.88±0.2°, 19.36±0.2°, 19.60±0.2°, 19.80±0.2°, 21.37±0.2° and 22.65±0.2°;
2) the X-ray powder diffraction pattern of the crystal form V is substantially as shown in FIG.13;
3) the crystal form V has a DSC thermogram substantially as shown in FIG.14; and
4) the crystal form V has a TGA thermogram substantially as shown in FIG.15.

The present invention provides a crystal form V of lurbinectedin, having characteristic absorption peaks at diffraction angles 2θ of 6.35±0.2°, 8.01±0.2°, 9.72±0.2°, 11.25±0.2°, 11.78±0.2°, 14.23±0.2°, 14.77±0.2°, 17.04±0.2°, 20.42±0.2° and 22.65±0.2° in the X-ray powder diffraction pattern using a Cu-Kα radiation source. The present invention provides a crystal form V of lurbinectedin, having characteristic absorption peaks at diffraction angles 2θ of 6.35±0.2°, 7.22±0.2°, 8.01±0.2°, 9.72±0.2°, 10.18±0.2°, 11.25±0.2°, 11.78±0.2°, 12.75±0.2°, 13.82±0.2°, 14.23±0.2°, 14.77±0.2°, 15.04±0.2°, 15.74±0.2°, 17.04±0.2°, 17.88±0.2°, 19.36±0.2°, 19.60±0.2°, 19.80±0.2°, 20.42±0.2°, 21.37±0.2° and 22.65±0.2° in the X-ray powder diffraction pattern using a Cu-Kα radiation source.

Without limitation, in one specific embodiment of the present invention, the X-ray powder diffraction pattern of the crystal form V of lurbinectedin is as shown in FIG. 13.

Without limitation, in a specific embodiment of the present invention, the crystal form V of lurbinectedin provided by the present invention, when subjected to differential scanning calorimetry (DSC) with a heating rate of 10°C/min, exhibits an obvious peak between about 25-150°C with peak point of about 59°C and a spilt exothermic peak in the range of about 150-215°C; corresponding to thermogravimetric analysis (TGA), from room temperature to 105°C, the weight loss is about 4% or less; a gentle slope-like weight loss is observed from room temperature to 150°C, and the weight loss is about 7% or less, about 6% or less, about 5.5% or less, preferably about 5% or less, more preferably 4.5% or less. Decomposition and weight loss begin in the temperature range of 150-200°C, and in one specific embodiment the peak point is about 180°C. The DSC thermogram is as shown in FIG.14, and the TGA thermogram is as shown in FIG.15.

The present invention also provides a method for preparing the crystal form V of lurbinectedin, comprising the following steps:
1) adding lurbinectedin to a solvent and suspending or slurring at an appropriate temperature for a certain time;
2) filtering; and
3) drying at room temperature to obtain the crystal form V.

The mass-to-volume ratio of lurbinectedin to the solvent is 1:(12-90) g:mL,preferably 1:(20-30) g:mL.

The solvent can be a single solvent or a mixed solvent, including but not limited to acetone or a mixed solvent of acetone and alkane solvent, the alkane solvent is preferably one or more member selected from the group consisting of n-pentane, n-hexane, n-heptane, and n-octane, more preferably n-heptane; the solvent in step 1) is preferably acetone or a mixed solvent of acetone and n-heptane. The appropriate temperature can be selected according to conventional ranges in the art, preferably 20-25°C; the certain time of suspending or slurring is 12 hours-4 days, preferably 2-4 days, more preferably 4 days. The filtration is any of solid-liquid separation methods such as centrifugal filtration and pressure filtration. the mass-to-volume ratio of lurbinectedin to the ketones (such as acetone) can be selected according to conventional methods in the art, preferably 1 g : 12 mL - 1 g : 90 mL. In some embodiments of the present invention, the solvent in step 1) is a mixed solvent of acetone and n-heptane, and preferably, the volume ratio of acetone to n-heptane is 1:10-10:1, more preferably 1: 10-2: 1; the mass-to-volume ratio of lurbinectedin to the total volume of acetone and n-heptane is 1 g : 12 mL - 1 g : 90 mL. The drying is a drying at room temperature, preferably a blast drying at room temperature of 20-25°C. In the present invention, the term "substantially" used in the phrase "X-ray powder diffraction pattern is substantially as shown in FIG. 1" means that the exact position of the peak in attached figures should not be interpreted as an absolute value, because those skilled in the art know that the 2θ value of an X-ray powder diffraction pattern may generate errors due to different measurement conditions (such as the equipment and instruments used) and different samples. A measurement error of diffraction angle of X-ray powder diffraction pattern is 5% or less, and generally, a deviation of ±0.2° of the given value will be considered appropriate. It should also be understood that the relative strength of the peak may fluctuate depending on experimental conditions and sample preparation, such as the preferred orientation of particles in the sample. The use of automatic or fixed divergent slits will also affect the calculation of relative strength. The intensity shown in the XRPD curve included here is only illustrative and should not be used for absolute comparison.

In the present invention, the term "about" means a scope within ±10% of the given value is comprised.

The present invention also provides use of a polymorph, wherein the polymorph is one or more of crystal form I, crystal form II, crystal form III, crystal form IV and crystal form V, and the use is use of the polymorph in the manufacture of a medicament for treating metastatic small cell lung cancer in an adult patient who have progressed following prior platinum-based chemotherapy.

The positive progressive effects of the crystal form V provided by the present invention comprise:
(1) the preparation method of the invention realizes the advantages of simple operation, stable process and high yield, and effectively avoids the use of highly toxic reagents like chloroform and carbon tetrachloride; the yield also reaches 90%, the process is stable, the reaction solution does not need nitrogen protection and does not discolor, and white solid product can be obtained, with HPLC purity higher than 99.5%; and/or
(2) the purification method of the invention can effectively remove impurities, improve product purity, and has simple operation and high yield.

The present invention also provides a pharmaceutical composition, which comprises one or more of the crystal form I, crystal form II, crystal form III, crystal form IV, and crystal form V of lurbinectedin mentioned above, along with pharmaceutical acceptable excipients. The pharmaceutical composition is preferably a lyophilized powder for injection. More preferably, the lurbinectedin lyophilized formulation consists of 4 mg lurbinectedin, sucrose (800 mg), lactic acid (22.08 mg) and sodium hydroxide (5.12 mg). 4 mg of lyophilized formulation for injection is supplied as lyophilized powder in single-dose vials for reconstitution for intravenous use; wherein 4 mg of lurbinectedin raw material is provided by crystal form I, crystal form II, crystal form III, crystal form IV or crystal form V.

The present invention also provides use of the pharmaceutical composition in the manufacture of a medicament in treating metastatic small cell lung cancer in an adult patient who have progressed following prior platinum-based chemotherapy, the pharmaceutical composition comprising one or more of crystal form I, crystal form II, crystal form III, crystal form IV and crystal form V of lurbinectedin mentioned above. The present invention also provides a pharmaceutical composition, wherein the active pharmaceutical ingredient of lurbinectedin is prepared from the polymorph of lurbinectedin mentioned above.

The present invention also provides a pharmaceutical composition, wherein the active pharmaceutical ingredient of lurbinectedin is prepared from the polymorph of lurbinectedin mentioned above, the polymorph of lurbinectedin can be used in the manufacture of a medicament in treating metastatic small cell lung cancer in an adult patient who have progressed following prior platinum-based chemotherapy.

The inventors observed the appearance of electrostatic properties of a crystal form B and the crystal form I, II, III, IV, V, and finds that the crystal form I, II, III, IV, V of the present invention exhibit better performance compared to crystal form B. Specifically, it shows that crystal form B exhibits wall-hanging phenomenon when stored in a glass bottle, while crystal form I, II, III, IV, and V have no wall-hanging phenomenon or the phenomenon is not obvious; during the weighing, crystal form B shows sticking to the scoop and weighing paper, indicating an obvious electrostatic effect, whereas crystal form I, II, III, IV, and V do not show electrostatic effect or the effect is not obvious.

The beneficial technical effects of the present invention are as follows: the new crystal form I of lurbinectedin provided by the present invention has the advantages such as high purity, excellent stability and good solubility; and it does not present the filtration difficulties or electrostatic issues found in the prior art. The preparation method of the crystal form I of lurbinectedin is simple, the solvent is cheap and easily available, and the crystallization conditions are mild, making it suitable for large-scale industrial production.

The new crystal form I of lurbinectedin has good solubility, which can ensure the therapeutic effect of the drug while reducing the difficulty of the development of developing lyophilized formulation, thus facilitating industrial production. The new crystal form I of lurbinectedin has excellent stability, which can remains stable for at least 12 months, preferably for over 18 months, and more preferably for over 20 months, when stored at 2-8°C or -20°C. The good stability of the crystal form I makes it more controllable during the crystallization process, reducing the likelihood of mixed crystals, thereby ensuring consistent and controllable product quality. The new crystal form I of lurbinectedin is easy to filter, has good product shape, good product loose bulk density, and no electrostatic phenomenon.

The crystal form II and crystal form IV provided by the present invention are anhydrous form, which can remain stable under accelerated conditions for 10 days and long-term conditions for 120 days, having good physical and chemical stability, good relative crystallinity, and no electrostatic issues. Form III is a hydrate form, which can be stored stably at room temperature under accelerated conditions for 10 days and long-term conditions for 120 days, and it also has good relative crystallinity and no electrostatic issues. Form V is an acetone solvate crystal form which can be used to obtain crystal form IV upon removal of the acetone solvent. The crystal form V is a highly advantageous crystal form with a high purity, especially due to its superior ability to be purified and remove impurities. The present invention provides new crystal form I, crystal form II, crystal form III, crystal form IV and crystal form V, which provides more choices for pharmaceutical lurbinectedin.

### DESCRIPTION OF DRAWINGS

The drawings described here are used to provide a further understanding of the present invention and constitute a part of the present invention. The schematic embodiments of the present invention and descriptions are used to explain the present application and do not intend to constitute any inappropriate limitations on the present invention.
FIG.1 is the X-ray powder diffraction (XPRD) pattern of the crystal form I of lurbinectedin of the present invention.
FIG.2 is the differential scanning calorimetry-thermogravimetric analysis (DSC-TGA) thermogram of the crystal form I of lurbinectedin of the present invention.
FIG.3 is the overlaid XPRD pattern of crystal form I of lurbinectedin from three test groups under the stability condition of the present invention.
FIG.4 is the XPRD pattern of the crystal form II of lurbinectedin of the present invention.
FIG.5 is the DSC thermogram of the crystal form II of lurbinectedin of the present invention.
FIG.6 is the TGA thermogram of the crystal form II of lurbinectedin of the present invention.
FIG.7 is the XPRD pattern of the crystal form III of lurbinectedin of the present invention.
FIG.8 is the DSC thermogram of the crystal form III of lurbinectedin of the present invention.
FIG.9 is the TGA thermogram of the crystal form III of lurbinectedin of the present invention.
FIG.10 is the XPRD pattern of the crystal form IV of lurbinectedin of the present invention.
FIG. 11 is the DSC thermogram of the crystal form IV of lurbinectedin of the present invention.
FIG. 12 is the TGA thermogram of the crystal form IV of lurbinectedin of the present invention.
FIG.13 is the XPRD pattern of the crystal form V of lurbinectedin of the present invention.
FIG.14 is the DSC thermogram of the crystal form V of lurbinectedin of the present invention.
FIG.15 is the TGA thermogram of the crystal form V of lurbinectedin of the present invention.

### DETAILED DESCRIPTION OF INVENTION

The following specific embodiments are provided to further illustrate the present invention. It should be understood that these embodiments are only help to further understanding of the advantages and effects of the technical solution of the present invention and not to limit the scope of the present invention. The scope of protection of the invention is determined by the claims.

Experimental methods in the embodiments that are not specifically stated are typically conducted under conventional conditions or according to the conditions recommended by the manufacturer.

Unless otherwise indicated, raw materials or reagents used in the embodiments are commercially available.

Unless otherwise indicated, the reagents mentioned are used without purification. All solvents are purchased from commercial suppliers, such as Aldrich, and used without further treatment.

In the present invention, room temperature refers to 20-25°C; overnight refers to 12-16 h.

### Measurement method:

### X-Ray Powder Diffraction Analysis

The XPRD pattern was collected on a Bruker D8 Advance diffractometer, the parameters of the X-ray powder diffraction method were as follows: X-ray reflection parameters: Cu, Kα; tube voltage: 40 kilovolts (kV); tube current: 40 milliamps (mA); slit: primary optical path Soller slit 2.5°, secondary optical path Soller slit 2.5°; scanning mode: continuous-scan; step angle: 0.02°, sampling time: 0.1s/step; scanning range: 3.0°-40.0°.

### (2)Thermogravimetric Analysis

The TGA thermogram was collected on a TA Instruments Discovery TGA 55, the parameters of the thermogravimetric analysis method were as follows: scanning rate: 10°C/min; protective gas: nitrogen.

### (3) Differential Scanning Calorimetry

The DSC thermogram was collected on a TA Instruments Discovery DSC250, test conditions: heating from 25°C to 250°C at a rate of 10°C/min; detection environment conditions: room temperature: 21°C.

### Example 1

The preparation method of lurbinectedin: lurbinectedin may be prepared according to the preparation method provided in the experiment part of "A Scalable Total Synthesis of the Antitumor Agents Et-743 and Lurbinectedin", Ma Dawei et al. Angew Chem Int Ed Engl. 2019 Mar18;58(12):3972-3975; or
is prepared according to the following method.

### Preparation method of lurbinectedin:

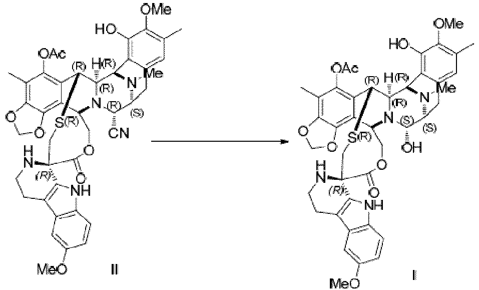

Deionized water (500 mL) was added to a 3 L three-necked flask, controlled the temperature at 0-25°C, added dimethyl sulfoxide (DMSO, 500 mL) dropwise, under the protection of argon, controlled the temperature at 10-25°C, and the cyanide of formula II (10 g, 12.6 mmol) was added to the reaction flask, then a solution of AgNO₃ (21.4 g, 126 mmol) in water (100 mL) was added dropwise to the reaction flask. After the dropwise addition, the reaction solution was reacted at 20-25°C under the protection of argon for 4-16 h, after the thin-layer chromatography (TLC) detection reaction is finished, the reaction was stopped and quenched by adding 1000 mL of dichloromethane and 500 mL of saturated sodium bicarbonate aqueous solution, controlled the temperature at 0-10°C and stirred for 30 min, filtered, collected the filtrate, layered, and decompressed to condense the organic layer to obtain 10 g of off-white solid. The molar yield of crude product: 100%.

### Example 2

### Preparation of crystal form I of Lurbinectedin:

The solid obtained in Example 1 was added to dichloromethane (300 mL), stirred to dissolve, then cooled to 0-10°C, added 1mol/L hydrochloric acid (26 mL) solution to adjust pH=1-2, stirred and separated the liquid. Aqueous phase was washed with dichloromethane (260 mL*2) at 0-10°C for 10 min with stirring, separated the liquid, cooled the aqueous phase to 0-10°C, added 6-10% sodium bicarbonate aqueous solution dropwise to adjust pH=9-10, and extracted with dichloromethane (260 mL*2) twice, combined two organic phases and washed with water (260 mL) once, the organic phase was dried with anhydrous sodium sulfate (20 g), and filtered by suction. The filtrate was distilled under reduced pressure at 20±3°C to obtain the concentrate lurbinectedin (purity 98.20%).

Taken 15.0 g of lurbinectedin (purity 98.20%) and added acetonitrile (100 mL) to dissolve, added water (100 mL) dropwise, and stirred at 10-25°C for 0.5 h. After suction filtration, the filter cake was dried to obtain product of the crystal form I of lurbinectedin: 7.8 g, white solid, yield: 78.9% (LCMS: [MS+1]=785.3), HPLC purity 99.91%; the XRD pattern is shown in FIG.1, the XRPD diffraction peak data are shown in Table 1, the DSC-TGA thermogram of Form I of lurbinectedin is shown in FIG.2, when thermogravimetric analysis is performed, the mass loss is about 1.25% from room temperature to 150°C; it start to lose weight obviously in the temperature range of 150-200°C, in one specific embodiment, the peak point is about 181°C where the weight loss is obvious, and the weight loss between 150-250°C is about 16.67% of the mass.

**Table 1 XRPD diffraction peak data of crystal form I of lurbinectedin**

| No. | 2θ Angle (° ) | Relative Intensity (%) | No. | 2θ Angle (°) | Relative Intensity (%) |
|---|---|---|---|---|---|
| 1 | 4.55 | 72.3 | 16 | 16.15 | 2.3 |
| 2 | 6.16 | 21.3 | 17 | 16.79 | 4.0 |
| 3 | 7.61 | 32.1 | 18 | 18.36 | 5.4 |
| 4 | 8.94 | 11.6 | 19 | 19.09 | 10.3 |
| 5 | 9.17 | 48.6 | 20 | 19.96 | 6.4 |
| 6 | 9.54 | 100.0 | 21 | 20.53 | 2.7 |
| 7 | 9.89 | 25.8 | 22 | 21.17 | 1.1 |
| 8 | 10.84 | 16.4 | 23 | 22.78 | 6.2 |
| 9 | 10.84 | 16.4 | 24 | 24.04 | 3.2 |
| 10 | 11.16 | 2.8 | 25 | 24.56 | 3.2 |
| 11 | 12.15 | 17.5 | 26 | 25.51 | 2.2 |
| 12 | 12.38 | 17.6 | 27 | 25.91 | 2.3 |
| 13 | 13.00 | 2.6 | 28 | 26.40 | 2.0 |
| 14 | 13.98 | 15.7 | 29 | 27.09 | 1.9 |
| 15 | 14.80 | 10.7 | - | - | - |

### Example 3

Taken 15.0 g of lurbinectedin (purity 98.20%) and added 140 mL of acetonitrile, controlled the temperature at 0-25°C, and stirred for 10 min, added 70 mL of water dropwise, cooled to 10-15°C and crystallization, heat-preserved for 1-2 hours to continue crystallization, filtered, the filter cake was rinsed with 20 mL of water, and the filter cake was vacuum-dried to obtain 13.5 g of the crystal form I of lurbinectedin (purity 99.58%).

### Example 4

Taken 15.0 g of lurbinectedin (purity 98.20%) and added 150 mL of acetonitrile, controlled the temperature at 10-25°C, and stirred for 10 min, added 100 mL of water dropwise, cooled to 10-15°C and crystallization, heat-preserved for 0.5-0.8 hour to continue crystallization, filtered, the filter cake was rinsed with 20 mL of water, and the filter cake was vacuum-dried to obtain 13.0 g of the crystal form I of lurbinectedin (purity 99.48%).

### Example 5

Taken 15.0 g of lurbinectedin (purity 98.20%) and added 140 mL of acetone, controlled the temperature at 15-20°C, and stirred for 10 min, added 140 mL of water dropwise, cooled to 10-15°C and crystallization, heat-preserved for 0.5-1 hour to continue crystallization, filtered, the filter cake was rinsed with 20 mL of water, and the filter cake was vacuum-dried to obtain 12.8 g of the crystal form I of lurbinectedin (purity 99.50%).

### Example 6

Taken 15.0 g of lurbinectedin (purity 98.20%) and added 150 mL of acetone, controlled the temperature at 20-25°C, and stirred for 10 min, added 100 mL of water dropwise, cooled to 10-15°C and crystallization, heat-preserved for 1-2 hours to continue crystallization, filtered, the filter cake was rinsed with 30 mL of water, and the filter cake was vacuum-dried to obtain 13.8 g of the crystal form I of lurbinectedin (purity 99.32%).

### Example 7

Taken 15.0 g of lurbinectedin (purity 98.20%) and added 100 mL of acetonitrile and 110 mL of water, controlled the temperature at 10-25°C, stirred for 0.5-1 hour and crystallization, filtered, the filter cake was rinsed with 40 mL of water, and the filter cake was vacuum-dried to obtain 13.0 g of the crystal form I of lurbinectedin (purity 99.41%).

### Example 8

### Stability test of crystal form I of lurbinectedin

Stability: Group 1, crystal form I at the initial moment; Group 2, kept in dark, sealed and stored at 2-8°C for 12 months; Group 3, kept in dark, sealed and stored at -20°C for 20 months; samples from each group were taken for XRPD determination to compare the crystal form, and the purity was measured by high performance liquid chromatography (HPLC) to compare the chemical purity, the results are shown in Table 2.

**Table 2 Stability test results of the crystal form I of lurbinectedin**

| | Group 1 initial moment | Group 2 2-8°C for 12 months | Group 3 -20°C for 20 months |
|---|---|---|---|
| HPLC purity | 99.91% | 99.68% | 99.65% |
| Crystal form of lurbinectedin | crystal form I | crystal form I (unchanged) | crystal form I (unchanged) |

From the stability test results of lurbinectedin crystal form I shown in Table 2, it can be observed that crystal form I of the present invention exhibits excellent stability, after being stored at -20°C in the dark and sealed, and at 2-8°C in the dark and sealed, the product purity remained above 99%, and the crystal form I remained unchanged, these stability results indicating its excellent stability. The overlaid XPRD pattern of crystal form I of lurbinectedin from three test groups under the stability condition are shown in FIG.3, the results prove that these three groups of crystal form are all crystal form I, and exhibit no polymorphic transformation phenomenon.

### Example 9 Preparation of crystal form II of lurbinectedin

Taken 150 mg of lurbinectedin (purity 99.36%) and added 2.0 mL of acetonitrile to obtain a suspension, controlled the temperature at 20-25°C, stirred for 12 h, filtered, the solid was vacuum-dried to obtain 136.9 mg of the crystal form II of lurbinectedin (purity 99.59%).

### Example 10 Preparation of crystal form II of lurbinectedin

Taken 20 mg of Lurbinectedin (purity 99.36%) and added 0.5 mL of acetonitrile to obtain a suspension, controlled the temperature at 45-50°C, stirred for 24 h, centrifuged, the solid was vacuum-dried to obtain 18.8 mg of the crystal form II of lurbinectedin (purity 99.58%).

### Example 11 Preparation of crystal form II of lurbinectedin

Taken 10 mg of Lurbinectedin (purity 99.36%) and added 0.4 mL of dichloromethane, controlled the temperature at 20-25°C to dissolve and clarification, filtered, 3.0 mL of acetonitrile was added to the mother liquor, after precipitated solid, stirred for about 10 min at room temperature, centrifuged, the solid was vacuum-dried to obtain 9 mg of the crystal form II of lurbinectedin (purity 99.54%).

### Example 12 Preparation of crystal form II of lurbinectedin

Taken 20 mg of Lurbinectedin (purity 99.36%) and added 0.2 mL of acetonitrile and 0.2 mL of ethyl acetate to obtain a suspension, controlled the temperature at 45-50°C, stirred for 36 h, centrifuged, the solid was vacuum-dried to obtain 18.5 mg of the crystal form II of lurbinectedin (purity 99.60%).

### Example 13 Preparation of crystal form II of lurbinectedin

Taken 10 mg of lurbinectedin (purity 99.36%) and added 0.4 mL of dichloromethane, dissolved to clarification with ultrasound and filtered, controlled the temperature at 20-25°C, 3.0 mL of acetonitrile was added to the mother liquor, stirred for about 10 min, centrifuged, the solid was vacuum-dried to obtain 19.1 mg of the crystal form II of lurbinectedin (purity 99.58%).

### Example 14 Preparation of crystal form III of lurbinectedin

Taken 150 mg of lurbinectedin (purity 99.36%) and added 10 mL of THF, dissolved to clarification with ultrasound, filtered, 25 mL of water was slowly added to the mother liquor, precipitated solid, controlled the temperature at 20-25°C, stirred for about 10 min, filtered, the filter cake was dried at room temperature to obtain 153.0 mg of the crystal form III of lurbinectedin (purity 99.61%).

### Example 15 Preparation of crystal form III of lurbinectedin

Taken 10 mg of lurbinectedin (purity 99.36%) and added 0.8 mL of THF, dissolved to clarification with ultrasound, filtered, 2.0 mL of water was added to the mother liquor, precipitated solid, controlled the temperature at 20-25°C, stirred for about 10 min, then centrifuged the solid, dried at room temperature to obtain 10 mg of the crystal form III of lurbinectedin (purity 99.65%).

### Example 16 Preparation of crystal form III of lurbinectedin

Taken 20 mg of lurbinectedin (purity 99.36%) and added a mixed solvent of 0.2 mL of water and 0.2 mL of 1,4-dioxane to obtain a mixed suspension, stirred for 4 days at 50°C, filtered, dried at room temperature to obtain 20 mg of the crystal form III of lurbinectedin (purity 99.60%).

### Example 17 Preparation of crystal form III of lurbinectedin

Taken 20 mg of lurbinectedin (purity 99.36%) and added a mixed solvent of 0.2 mL of ethyl acetate and 0.2 mL of isopropanol to obtain a mixed suspension, stirred for 4 days at 25°C, filtered, dried at room temperature to obtain 19.8 mg of the crystal form III of lurbinectedin (purity 99.62%).

### Example 18 Preparation of crystal form IV of lurbinectedin

Take 150 mg of lurbinectedin (purity 99.36%) and added a mixed solvent of 1.8 mL of acetone and 0.9 mL of n-heptane, slurried overnight at room temperature, and vacuum-dried at 40°C overnight to obtain crystal form IV, weighed 130.8 mg (purity 99.90%).

### Example 19 Preparation of crystal form IV of lurbinectedin

Taken 20 mg of lurbinectedin (purity 99.36%) and added 0.5 mL of acetone to obtain a mixed suspension, controlled the temperature at 20-25°C, stirred for 4 days, centrifuged, the solid was vacuum-dried at room temperature to obtain 18.0 mg of the crystal form IV of lurbinectedin (purity 99.86%).

### Example 20 Preparation of crystal form IV of lurbinectedin

Taken 20 mg of lurbinectedin (purity 99.36%) and added 0.4 mL of acetone and 0.2 mL of n-heptane, controlled the temperature at 20-25°C, stirred for 4 days, centrifuged, the solid was vacuum-dried at room temperature to obtain 18.4 mg of the crystal form IV of lurbinectedin (purity 99.92%).

### Example 21 Preparation of crystal form IV of lurbinectedin

Taken 20 mg of lurbinectedin (purity 99.36%) and added 1.8 mL of acetone and 0.9 mL of n-heptane, controlled the temperature at 20-25°C, stirred for 1 day, centrifuged, the solid was vacuum-dried at room temperature to obtain 18.5 mg of the crystal form IV of lurbinectedin (purity 99.92%).

### Example 22 Preparation of crystal form V of lurbinectedin

Take 20mg of lurbinectedin (purity 99.36%) and added 0.4mL of acetone to obtain a suspension, stirred at 25°C for 4 days, filtered, and dried at room temperature to obtain 19.0 mg of the crystal form V of lurbinectedin (purity 99.93%).

### Example 23 Preparation of crystal form V of lurbinectedin

Take 20mg of lurbinectedin (purity 99.36%) ,added a mixed solvent of 0.2 mL of n-heptane and 0.4 mL of acetone to obtain suspension, stirred at room temperature(20-25°C) for 4 days, filtered, and dried at room temperature to obtain 18.8 mg of the crystal form V of lurbinectedin (purity 99.92%).

### Example 24 Stability test of crystal form II, crystal form III and crystal form IV of lurbinectedin

Stability: Group 1, crystal form II, crystal form III, crystal form IV and crystal form B at the initial moment; Group 2, 25°C-60% relative humidity (RH) for 5 days; Group 3, 25°C-60% RH for 10 days; Group 4, 40°C-75% RH for 5 days; Group 5, 40°C-75% RH for 10 days; Group 6, stored in a dark, sealed environment at -20°C for 120 days; samples from each group were taken for XRPD determination to compare the crystal form, and the purity was measured by HPLC to compare the chemical purity, the results are shown in Table 3.

**Table 3 Stability test results of the crystal form II, III and IV of lurbinectedin**

| Crystal form | Example 9 Crystal form II | Example14 Crystal form III | Example 18 Crystal form IV | WO2021099635 Example 2 Crystal form B |
|---|---|---|---|---|
| Group 1, HPLC purity (0 day) | 99.59% | 99.61% | 99.90% | 99.62% |
| Group 2, HPLC purity (25°C/60% RH-5 days) | 99.40% | 99.48% | 99.86% | 99.44% |
| Group 3, HPLC purity (25°C/60% RH-10 days) | 99.41% | 99.42% | 99.74% | 99.05% |
| Group 4, HPLC purity (40°C/75% RH-5 days) | 99.41% | 99.48% | 99.79% | 99.13% |
| Group 5, HPLC purity (40°C/75% RH-10 days) | 99.41% | 99.37% | 99.78% | 99.24% |
| Group 6, HPLC purity (sealed and stored at -20°C for 120 days) | 99.58% | 99.60% | 99.91% | 99.60% |
| XRPD | The crystal form of Form II remains unchanged for 10 days under long-term and accelerated conditions; the crystal form remains unchanged at -20°C for 120 days. | The crystal form of Form III remains unchanged for 10 days under long-term and accelerated conditions; the crystal form remains unchanged at -20°C for 120 days. | The crystal form of Form IV remains unchanged for 10 days under long-term and accelerated conditions; the crystal form remains unchanged at -20°C for 120 days. | The crystal form of Form B remains unchanged for 10 days under long-term and accelerated conditions; the crystal form remains unchanged at -20°C for 120 days. |

From the stability test results of lurbinectedin crystal form II shown in Table 3, it can be observed that crystal form II of the present invention exhibits excellent physical and chemical stability, after being stored for 10 days under conditions of 25°C/60% RH and 40°C/75% RH, the product purity remained above 99%, and the crystal form II remained unchanged; when sealed and stored at -20°C for 120 days, the crystal form remained unchanged, with no polymorphic transformation observed, and the purity remained above 99%.

From the stability test results of lurbinectedin crystal form **III** shown in Table 3, it can be observed that crystal form **III** of the present invention exhibits excellent physical and chemical stability, after being stored for 10 days under conditions of 25°C/60% RH and 40°C/75% RH, the product purity remained above 99%, and the crystal form **III** remained unchanged; when sealed and stored at -20°C for 120 days, the crystal form remained unchanged, with no polymorphic transformation observed, and the purity remained above 99%.

From the stability test results of lurbinectedin crystal form IV shown in Table 3, it can be observed that crystal form IV of the present invention exhibits excellent physical and chemical stability, after being stored for 10 days under conditions of 25°C/60% RH and 40°C/75% RH, the product purity remained above 99%, and the crystal form IV remained unchanged; when sealed and stored at -20°C for 120 days, the crystal form remained unchanged, with no polymorphic transformation observed, and the purity remained above 99%.

From the stability test results of lurbinectedin crystal form B shown in Table 3,, it can be observed that after 10 days under long-term and accelerated conditions, the crystal form of Form B remained unchanged, indicating that its physical stability is excellent, whereas its purity decreased, indicating that its chemical stability is relatively poor. When sealed and stored at -20°C for 120 days, the crystal form remained unchanged, with no polymorphic transformation observed, and the purity remained above 99%.

The above results show that the crystal form II, III and IV of lurbinectedin of the present application have excellent stability, these crystal forms can remain stable for at least 10 days when placed at room temperature and at least 120 days at -20°C.

It should be noted that the documents cited in the application are incorporated herein by reference in their entirety, and will not be described in detail herein.

The aforementioned examples are only preferred embodiments of the present invention and are not intended to limit the scope of invention. Any modification, equivalent replacement, improvement and the like made within the spirit and principle of the present invention shall be included in the protection scope of the present invention.

## Claims

1. A polymorph of the compound represented by Formula (I), wherein the polymorph is selected from the group consisting of crystal form I, crystal form II, crystal form III, crystal form IV, and crystal form V, 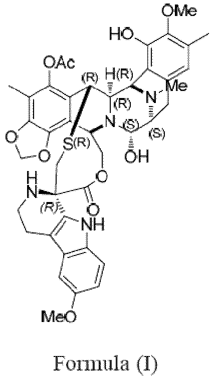
wherein the crystal form I has characteristic absorption peaks at diffraction angles 2θ of 4.55±0.2°, 6.16±0.2°, 7.61±0.2°, 9.17±0.2°, 9.54±0.2°, and 9.89±0.2° in an X-ray powder diffraction pattern;
the crystal form II has characteristic absorption peaks at diffraction angles 2θ of 4.56±0.2°, 9.26±0.2°, 9.54±0.2°, 9.93±0.2°, 13.99±0.2°, 18.46±0.2° and 19.02±0.2° in an X-ray powder diffraction pattern;
the crystal form III has characteristic absorption peaks at diffraction angles 2θ of 4.82±0.2°, 8.75±0.2°, 10.13±0.2°, 10.51±0.2°, 13.23±0.2° and 17.98±0.2° in an X-ray powder diffraction pattern;
the crystal form IV has characteristic absorption peaks at diffraction angles 2θ of 5.57±0.2°, 7.18±0.2°, 8.70±0.2°, 11.20±0.2°, 11.65±0.2° and 13.63±0.2° in an X-ray powder diffraction pattern; and
the crystal form V has characteristic absorption peaks at diffraction angles 2θ of 6.35±0.2°, 8.01±0.2°, 9.72±0.2°, 14.23±0.2°, 17.04±0.2° and 20.42±0.2° in an X-ray powder diffraction pattern.

2. The polymorph according to claim 1, wherein the crystal form I has one or more characteristics selected from the group consisting of:
1) the crystal form I has characteristic absorption peaks at diffraction angles 2θ of 4.55±0.2°, 6.16±0.2°, 7.61±0.2°, 9.17±0.2°, 9.54±0.2° and 9.89±0.2° in the X-ray powder diffraction pattern, and has one or more of the characteristic peaks at diffraction angles 2θ of: 8.94±0.2°, 10.84±0.2°, 11.16±0.2°, 12.15±0.2°, 12.38±0.2°, 13.00±0.2°, 13.98±0.2°, 14.80±0.2°, 15.24±0.2°, 16.15±0.2°, 16.79±0.2°, 18.36±0.2°, 19.09±0.2°, 19.96±0.2°, 20.53±0.2°, 21.17±0.2°, 22.78±0.2°, 24.04±0.2°, 24.56±0.2°, 25.51±0.2°, 25.91±0.2°, 26.40±0.2° and 27.09±0.2°;
2) the X-ray powder diffraction pattern of the crystal form I is substantially as shown in FIG.1; and
3) the crystal form I has a DSC-TGA thermogram substantially as shown in FIG.2.

3. The polymorph according to claim 1, wherein the crystal form II has one or more characteristics selected from the group consisting of:
1) the crystal form II has characteristic absorption peaks at diffraction angles 2θ of 4.56±0.2°, 9.26±0.2°, 9.54±0.2°, 9.93±0.2°, 13.99±0.2°, 18.46±0.2° and 19.02±0.2° in the X-ray powder diffraction pattern, and has one or more of the characteristic peaks at diffraction angles 2θ of: 11.20±0.2°, 12.13±0.2°, 12.34±0.2°, 12.99±0.2°, 15.27±0.2°, 16.19±0.2°, 16.80±0.2°, 17.41±0.2°, 20.01±0.2°, 20.26±0.2°, 22.78±0.2°, 24.07±0.2°, 24.51±0.2°, 25.02±0.2° and 25.49±0.2°;
2) the X-ray powder diffraction pattern of the crystal form II is substantially as shown in FIG.4;
3) the crystal form II has a DSC thermogram substantially as shown in FIG.5; and
4) the crystal form II has a TGAthermogram substantially as shown in FIG.6.

4. The polymorph according to claim 1, wherein the crystal form III has one or more characteristics selected from the group consisting of:
1) the crystal form III has characteristic absorption peaks at diffraction angles 2θ of 4.82±0.2°, 8.75±0.2°, 10.13±0.2°, 10.51±0.2°, 13.23±0.2° and 17.98±0.2° in the X-ray powder diffraction pattern, and has one or more of the characteristic peaks at diffraction angles 20 of: 5.22±0.2°, 6.15±0.2°, 7.62±0.2°, 7.83±0.2°, 9.02±0.2°, 9.62±0.2°, 11.57±0.2°, 11.91±0.2°, 12.42±0.2°, 14.12±0.2°, 14.82±0.2°, 15.85±0.2°, 16.55±0.2°, 17.18±0.2°, 18.8±0.2°, 19.21±0.2°, 19.41±0.2°, 20.65±0.2°, 23.10±0.2°, 23.34±0.2°, 24.17±0.2°, 25.26±0.2° and 25.51±0.2°;
2) the X-ray powder diffraction pattern of the crystal form III is substantially as shown in FIG.7;
3) the crystal form III has a DSC thermogram substantially as shown in FIG.8; and
4) the crystal form III has a TGA thermogram substantially as shown in FIG.9.

5. The polymorph according to claim 1, wherein the crystal form IV has one or more characteristics selected from the group consisting of:
1) the crystal form IV has characteristic absorption peaks at diffraction angles 5.57±0.2°, 7.18±0.2°, 8.70±0.2°, 11.20±0.2°, 11.65±0.2° and 13.63±0.2° in the X-ray powder diffraction pattern, and has one or more of the characteristic peaks at diffraction angles 20 of: 7.91±0.2°, 9.56±0.2°, 10.72±0.2°, 12.44±0.2°, 12.63±0.2°, 14.40±0.2°, 15.58±0.2°, 15.90±0.2°, 16.23±0.2°, 16.46±0.2°, 17.18±0.2°, 18.47±0.2°, 19.18±0.2°, 20.28±0.2°, 20.73±0.2°, 21.70±0.2°, 23.00±0.2°, 23.18±0.2°, 24.86±0.2° and 27.14±0.2°;
2) the X-ray powder diffraction pattern of the crystal form IV is substantially as shown in FIG.10;
3) the crystal form IV has a DSC thermogram substantially as shown in FIG.11; and
4) the crystal form IV has a TGA thermogram substantially as shown in FIG.12.

6. The polymorph according to claim 1, wherein the crystal form V has one or more characteristics selected from the group consisting of:
1) the crystal form V has characteristic absorption peaks at diffraction angles 2θ of 6.35±0.2°, 8.01±0.2°, 9.72±0.2°, 14.23±0.2°, 17.04±0.2° and 20.42±0.2° in the X-ray powder diffraction pattern, and has one or more of the characteristic peaks at diffraction angles 20 of: 7.22±0.2°, 10.18±0.2°, 11.25±0.2°, 11.78±0.2°, 12.75±0.2°, 13.82±0.2°, 14.77±0.2°, 15.04±0.2°, 15.74±0.2°, 17.88±0.2°, 19.36±0.2°, 19.60±0.2°, 19.80±0.2°, 21.37±0.2° and 22.65±0.2°;
2) the X-ray powder diffraction pattern of the crystal form V is substantially as shown in FIG.13;
3) the crystal form V has a DSC thermogram substantially as shown in FIG.14; and
4) the crystal form V has a TGA thermogram substantially as shown in FIG.15.

7. A method for preparing the polymorph according to claim 1, wherein the method is:
Method 1: a method for preparing the crystal form I, comprising the step of:
1-1) dissolving lurbinectedin in a good solvent at an appropriate temperature, then adding an anti-solvent dropwise until turbidity appears, followed by stirring at a certain temperature to induce crystallization, filtering, and drying to obtain the crystal form I; or
1-2) dissolving lurbinectedin in a mixed solvent of a good solvent and an anti-solvent at an appropriate temperature, stirring to induce crystallization, and filtering and drying the resultant to obtain the crystal form I; and
preferably, the volume ratio of the good solvent to the anti-solvent in step 1-1) or 1-2) is 1:3 to 3:1, more preferably 1:1 to 2:1; and/or, the mass-to-volume ratio of lurbinectedin to the solvent is 1 g : 5 mL to 1 g : 30 mL, preferably 1 g : 10 mL to 1 g : 20 mL, wherein the solvent is a generic term for the good solvent and the anti-solvent;
preferably, the good solvent in step 1-1) or 1-2) is an aprotic polar solvent, which is selected from the group consisting of N-methyl pyrrolidone, nitrile solvent, ketone solvent, and a combination thereof; preferably, the nitrile solvent is selected from the group consisting of acetonitrile, propionitrile, butyronitrile and isobutyronitrile; preferably, the ketone solvent is selected from the group consisting of acetone, butanone, and 2-pentanone; and the anti-solvent is selected from the group consisting of water, diethyl ether, isopropyl ether, methyl tert-butyl ether, and a combination thereof; and/or
preferably, the appropriate temperature in step 1-1) or 1-2) is 10-25°C; and/or the certain temperature in step 1-1) is 10-15°C.

8. A method for preparing the polymorph according to claim 1, wherein the method is selected from the group consisting of:
Method 2: a method for preparing the crystal form II, comprising the step of:
2-1) adding lurbinectedin to a solvent and suspending at an appropriate temperature for a certain time, followed by filtering and drying at a certain temperature to obtain the crystal form II; or
2-2) dissolving lurbinectedin in a good solvent at an appropriate temperature, adding an anti-solvent to the solution thus obtained, stirring to induce crystallization at a certain temperature, and filtering and drying the resultant to obtain the crystal form II;
preferably, in step 2-1), the solvent is selected from the group consisting of nitriles, esters, chlorinated alkanes, ethers, and a combination thereof; more preferably selected from the group consisting of acetonitrile, dichloromethane, and a mixed solvent of acetonitrile and ethyl acetate; the appropriate temperature is 20-50°C; the time of suspension is 12 hours-4 days, preferably 1-4 days, and more preferably 4 days; the filtration is a centrifugal filtration or a vacuum filtration; the drying is a vacuum drying or a blast drying conducted at a temperature of 20-50°C, more preferably a vacuum drying at 40-50°C; the mass-to-volume ratio of lurbinectedin to the solvent is 40 : 1 mg : mL - 75 : 1 mg : mL; and/or
preferably, in step 2-2), the good solvent is a haloalkane solvent, more preferably dichloromethane or chloroform; the anti-solvent is a nitrile solvent, preferably acetonitrile; the appropriate temperature is 20-25°C; the certain temperature is 15-35°C, preferably 20-25°C; the volume ratio of the good solvent to the anti-solvent is 1:(6-9); the mass-to-volume ratio of lurbinectedin to the total volume of the good solvent and the anti-solvent is 1:(2-4) mg : mL;
Method 3: a method for preparing the crystal form III, comprising the step of:
3-1) dissolving lurbinectedin in a good solvent at an appropriate temperature, adding an anti-solvent to the solution thus obtained, stirring to induce crystallization, and filtering and drying the resultant at room temperature to obtain the crystal form III; or
3-2) adding lurbinectedin to a mixed solvent at an appropriate temperature, stirring to induce crystallization, and filtering and drying the resultant to obtain the crystal form III;
wherein, in step 3-1), the good solvent is preferably an aprotic polar solvent, which is preferably an ether solvent, more preferably tetrahydrofuran; the anti-solvent is preferably water, an alkane solvent, or a mixture thereof, and the alkane solvent is preferably selected from the group consisting of n-pentane, n-hexane, n-heptane, n-octane, and a combination thereof, more preferably n-heptane;
preferably, the volume ratio of the good solvent to the anti-solvent is 1:10-10:1, more preferably 1:2-1:10;
preferably, the mass-to-volume ratio of lurbinectedin to the good solvent is 1 g : 60 mL - 1 g : 500 mL, more preferably 1 g : 60 mL - 1 g : 200 mL; and/or the appropriate temperature is 20-25°C;
in step 3-2), the mixed solvent is preferably a mixed solvent of ethers and water, or a mixed solvent of esters and an alcohols, more preferably a mixed solvent of tetrahydrofuran and water, a mixed solvent of dioxane and water, or a mixed solvent of ethyl acetate and isopropanol; in the mixed solvent of ethers and water, the volume ratio of ethers to water is 1:(0.8-1.2); in the mixed solvent of esters and alcohols, the volume ratio of esters to alcohols is 1: (0.8-1.2); and/or
preferably, the appropriate temperature is 25-50°C; the mass-to-volume ratio of lurbinectedin to the mixed solvent is 1:(18-22) g:mL;
Method 4: a method for preparing the crystal form IV, comprising the step of:
4-1) adding lurbinectedin to a solvent and suspending or slurring at an appropriate temperature for a certain time, followed by filtering and vacuum drying to obtain the crystal form IV;
the solvent of step 4-1) is selected from the group consisting of ketones, esters, ethers, alcohols, alkanes, water, and a combination thereof, preferably selected from the group consisting of ketones and a mixed solvent of ketones with esters, ethers, alcohols, alkanes or water; preferably the ketones is acetone, the esters is one or more member selected from ethyl acetate and isopropyl acetate; the ethers is one or more member selected from methyl tert-butyl ether and tetrahydrofuran; the alcohols is one or more member selected from methanol, ethanol, and isopropanol; the alkanes is one or more member selected from n-heptane, n-hexane, cyclohexane and methyl cyclohexane;
the appropriate temperature is 20-25°C; preferably, the suspending or slurring is performed by adding lurbinectedin to acetone and suspending or slurring at 20-25°C, or by adding lurbinectedin to a mixed solvent of acetone/n-heptane in a volume ratio of (1-3):1 and suspending or slurring at 20-25°C; preferably, the time of suspending or slurring is 1 hour - 4 days, preferably 12 hours - 4 days, more preferably 1-4 days, and further preferably 4 days; the drying is vacuum drying, preferably vacuum drying at 40-50°C; and/or
the mass-to-volume ratio of lurbinectedin to the solvent is 1:(10-150) g:mL; and
Method 5: a method for preparing the crystal form V, comprising the step of:
5-1) adding lurbinectedin to a solvent and suspending or slurring at an appropriate temperature for a certain time, followed by filtering and drying at room temperature to obtain the crystal form V;
the solvent of step 5-1) is selected from the group consisting of acetone and a mixed solvent of acetone and an alkane solvent, wherein the alkane solvent is preferably selected from the group consisting of n-pentane, n-hexane, n-heptane, n-octane, and a combination thereof, more preferably is n-heptane; preferably, the solvent is acetone or a mixed solvent of acetone and n-heptane; the appropriate temperature is 20-25°C, the certain time of suspending or slurring is 12 hours - 4 days, preferably 2-4 days, more preferably 4 days; and/or
the mass-to-volume ratio of lurbinectedin to the solvent is 1:(12-90) g:mL, preferably 1:(20-30) g:mL.

9. The method according to claim 8, wherein when the solvent of step 5-1) is an acetone solvent, the mass-to-volume ratio of lurbinectedin to acetone is 1 g : 12 mL - 1 g : 90 mL; when the solvent of step 5-1) is a mixed solvent of acetone and n-heptane, the volume ratio of acetone to n-heptane is 1:10-10:1, preferably 1:10-2:1, and the mass-to-volume ratio of lurbinectedin to the total volume of acetone and n-heptane is 1 g : 12 mL - 1 g : 90 mL; the drying at room temperature is a blast drying at room temperature of 20-25°C.

10. Use of the polymorph according to claim 1, wherein the polymorph is selected from the group consisting of crystal form I, crystal form II, crystal form III, crystal form IV, crystal form V, and a combination thereof, and the use is use of the polymorph in the manufacture of a medicament for treating metastatic small cell lung cancer in an adult patient who has progressed following prior platinum-based chemotherapy.

11. A pharmaceutical composition, wherein the pharmaceutical composition comprises the polymorph according to claim 1, which is selected from the group consisting of crystal form I, crystal form II, crystal form III, crystal form IV, crystal form V, and a combination thereof; and pharmaceutical acceptable excipients.

12. Use of the pharmaceutical composition according to claim 11 in the manufacture of a medicament for treating metastatic small cell lung cancer in an adult patient who has progressed following prior platinum-based chemotherapy.
